# DEMANDE DE BREVET EUROPEEN

(11) **EP 3 913 363 A1**
(43) Date de publication de la demande: **24.11.2021**
(21) Numéro de dépôt: 21173056.9
(22) Date de dépôt: 10.05.2021
(51) Int. Cl.: G01N 31/22, A61L 2/28, A61L 2/20

(54) **DISPOSITIF ET MÉTHODE DE VÉRIFICATION DES PROCESSUS D'ASSAINISSEMENT D'AMBIANCE DE LOCAUX**

(30) Priorité: 19.05.2020 MC 2700
(71) Demandeur: Moffa, Ricardo, 98000 Monaco (MC); De Rosa, Alfredo, 80131 Napoli (IT); De Rosa, Roberto, 80131 Napoli (IT)
(72) Inventeur: Moffa, Ricardo, 98000 Monaco (MC); De Rosa, Alfredo, 80131 Napoli (IT); De Rosa, Roberto, 80131 Napoli (IT)
(74) Mandataire: Mola, Edoardo

(57) **Abrégé**

Cette invention s'inscrit dans le cadre de la surveillance des processus d'assainissement des ambiances de locaux effectués avec des formulations basées sur le peroxyde d'hydrogène et d'autres peroxydes organiques, en particulier lorsqu'ils sont utilisés sous forme atomisée ou en forme vaporisé, spray. L'invention concerne un dispositif qui est placé dans la chambre ou l'environnement qui doit être aseptisé, et, avec des variations chromatiques, permet d'évaluer en temps réel si la quantité de peroxyde d'hydrogène, par unité de surface, est suffisante pour assurer la destruction totale du coronavirus en particulier et d'autres agents pathogènes en général. Le dispositif préserve le résultat chromatique analytique, et il est utilisé comme preuve attaché à la documentation des processus d'assainissement qui nécessitent une vérification comme en exemple dans l'assainissement de l'intérieur des véhicules de transport en particulier avions qu'entre les vols doivent être aseptisés avec des processus rapides, vérifies et documentés.

## Description

### DOMAINE TECHNIQUE

Cette invention s'inscrit dans le cadre de la surveillance des processus d'assainissement de l'environnement effectués avec des formulations basées sur le peroxyde d'hydrogène et d'autres peroxydes organiques, en particulier lorsqu' ils sont utilisés sous forme atomisée. L'invention est particulièrement opportune pour le contraste avec la pandémie du SARS- CoV-2(Coronavirus du syndrome respiratoire aigu 2) qui peut être effectivement mise en œuvre avec des mesures d'assainissement environnementale drastiques et systématiques, en particulier dans les transports et les environnements de travail. L'invention concerne un dispositif, qui est placé dans l'environnement pour être aseptisé, avec des variations chromatiques permet d'évaluer en temps réel si la quantité de joint de peroxyde par unité de surface est suffisante pour assurer la destruction totale du coronavirus. L'appareil préserve la recherche analytique pour la documentation rituelle et est particulièrement utile pour assainir l'intérieur de l'avion qui, entre les vols, doit être aseptisé par des processus rapides et documentés.

### Etat de la technique antérieure

Dans le Pandémie grave actuel du SARS-CoV-2, la nécessité de réactiver les activités de production, même si l'infection n'a pas encore été complètement éradiquée, implique une action généralisée d'assainissement des environnements, en particulier ceux du travail et des moyens de transport.

L'agent causal impliqué dans les contaminations actuelles est le coronavirus 2019 (COVID-19), le SARS-CoV-2 (genre: Beta coronavirus), qui appartient à la famille des coronaviridaes, une grande famille de virus à ARN à brin unique enveloppés dans un sens positif. Les coronavirus sont transmis dans la plupart des cas par des gouttelettes respiratoires et des contacts, mais d'autres modes de transmission ont également été relevé.

Les temps de survie et les conditions qui influent sur la viabilité du SARS-CoV-2 dans l'environnement sont actuellement mal compris. Selon des études évaluant la stabilité environnementale d'autres coronavirus, on estime que le coronavirus du syndrome respiratoire aigu sévère (SARS-CoV) peut survivre pendant plusieurs jours dans l'environnement et que le coronavirus lié au syndrome respiratoire du Moyen-Orient (MERS-CoV) peut résister à plus de 48 heures à la température ambiante moyenne (20 degrés Celsius) sur différentes surfaces.

En raison de la survie potentielle du virus dans l'environnement pendant plusieurs jours, les locaux et les zones potentiellement contaminés par le SARS-CoV-2 doivent être aseptises avant leur réutilisation, à l'aide de produits contenant des agents antimicrobiens connus pour être efficaces même contre les coronavirus. Certains des ingrédients actifs, par exemple. L'hypochlorure de sodium (contenue dans l'eau de Javel domestique) et l'éthanol sont largement disponibles dans les milieux non-santé et non-laboratoire. Différentes raisons telles que l'inflammabilité de l'éthanol et l'agression chimique de l'hypochlorate de sodium rendent ces produits impropres à l'assainissement d'espaces grands et structurellement complexes. Parmi les techniques d'assainissement l'une des plus efficaces est celle qui utilise des peroxydes, en particulier l'eau oxygénée (peroxyde d'hydrogène stabilisé; H₂O₂). En ce qui concerne les virus du SARS, certaines études (A. Schmidt, M.H. Wolff et 0.Weber, In Coronaviruses with Special Emphasis on First Insights Concerning SARS - Birkh-user Advances in Infectious Diseases, 2005) ont montré une désinfection efficace avec 5 000 ppm (0,5 %) H₂O₂, qui en moins de 3 minutes réduit le titre viral de 10.000 fois. Une autre étude (Anthony F. Henwood, Journal of Histotechnology, DOI: 10.1080/01478885.2020), décrit des résultats similaires sur l'activité virucide de H₂O₂ à 0,5%, après 1 minute d'exposition. Plus d'informations sur l'efficacité viruid de 0,5% H₂O₂ est rapportée dans cette étude par Navid et al. (Navid Omidbakhsh, BSc, ae Syed A. Sattar, PhDb, 2005) qui démontre une réduction de l'infectiosité de 10 000 fois, même en présence de sérum de 5 % (mélange de protéines) pour simuler la présence de fluides corporels.

L'efficacité des traitements de sanctification peroxydiques augmente fortement lorsque H₂O₂ est introduit dans les environnements pour être aseptisé sous forme atomisée (microparticules d'une taille allant de 0,3 à 0,5 microns). Cela augmente de façon exponentielle la zone de contact d'assainissement de l'environnement et amplifie l'action de désinfection, réduisant ainsi le temps de contact nécessaire pour assurer un assainissement optimal.

Le peroxyde atomisé se comporte comme une phase pseudo-gazeuse, qui se propage uniformément sur chaque centimètre carré de surface dans l'environnement et pour chaque volume d'air, sans générer d'humidité, de corrosion et de résidus. Dès que les radicaux ossidriliques hautement oxydants entrent en contact avec les membranes des micro- organismes et des virus, ils déterminent les processus chimiques oxydants qui modifient leur structure. Ces altérations entraînent la perte de fonctions de confinement et de biochimie des membranes, entraînent la destruction de l'organisme dont elles font partie. Le traitement avec H₂O₂ atomisé ne génère pas la formation de composés organiques volatils et sa dégradation est rapide et supérieure à 99, 99% dans un temps très court (20 minutes). Pour ces caractéristiques, l'atomisation de produits tels que H₂O₂ et d'autres peroxydes organiques, entraîne une augmentation de l'effet biocide d'environ 1 million de fois. La décomposition rapide des radicaux oxydants dans H₂O et O₂ rend le composé non toxique et non cancérogène pour le personnel d'assainissement, qui ne sont pas autorisés à être présents pendant l'atomisation. La très forte efficacité contre les virus et les bactéries permet l'utilisation de faibles concentrations de H₂O₂ dans les produits qui sont atomisés dans l'environnement, assurant l'absence de corrosivité sur les matériaux présents dans les locaux aseptisés.

En ce qui concerne l'utilisation du H₂O₂ dans le traitement d'environnements particuliers tels que celui à l'intérieur des aéronefs, qui doit être effectué rapidement et de manière documentable à la fin de chaque voyage, l'OMS dans son "Guide d'hygiène et d'hygiène dans l'aviation" (Organisation mondiale de la Santé, 2009) déclare que les désinfectants basés sur H₂O₂ contenant des additifs, tels que les tensioactives et la chélation, obtiennent d'excellents résultats. L'Autorité chinoise de l'aviation civile, sur la base des résultats et de l'expérience pendant l'épidémie de SARS-CoV-2, recommande également l'utilisation d'aéronefs préparés à base de H₂O₂ dans l'assainissement. La concentration de H₂O₂ ne devrait pas être supérieure à 3% et le temps de réaction devrait être d'environ 20 minutes. Actuellement, une soixantaine de formulations basées sur H₂O₂ sont disponibles sur le marché international, également en association avec d'autres composés, dont 26% ont été spécifiquement approuvés par l'Environmental Protection Agency (EPA) des États-Unis.

Dans les procédures d'assainissement avec des peroxydes dans les 'espaces publics, en particulier l'intérieur des véhicules de transport tels que les métros, les trains et les avions, il est important de documenter la qualité et l'exhaustivité du processus d'assainissement effectué.

Il est donc nécessaire de fournir une méthode pour une certification de l'efficacité de l'assainissement environnemental de la contamination par le SARS-CoV-2, qui ne nécessite pas d'instruments spéciaux, tels que la lecture immédiate/quantitative, et fournit une documentation expérimentale réservable.

### DESCRIPTION DE L'INVENTION

Ce brevet revendique un dispositif innovant pour la qualité et la désignation quantitative des personnes, en particulier lorsqu'ils sont atomisés, ce qui permet de vérifier l'efficacité du processus d'assainissement de grands environnements technologiquement complexes tels que l'intérieur des métros, des trains et des avions. L'appareil utilise des réactifs stables à température ambiante, il est rapide, sensible, spécifique, transportable, bon marché et non invasif. Le dispositif de l'invention est basé sur la formation d'un complexe coloré très spécifique et stable qui se forme entre l'iode et l'amylose.

L'Amylose est un polysaccaride soluble dans l'eau, formé par de longues chaînes linéaires de D-glucose, reliés entre eux par des liaisons glycosidique alpha 1-4, qui enveloppent en spirales formées par 6 molécules de glucose par tour. L'amylose a état sec est une poudre blanche qui, dans l'eau, provient de solutions incolores et transparentes. L'amilosius en présence de l'iion forme un complexe bleu provenant de l'insertion de l'ion triiodé dans la spirale de l'amilosius. Cette réaction est très spécifique car les molécules apparentées telles que l'amylopécine ne donnent pas cette réaction, qui pour sa spécificité et sa vitesse est déjà utilisée dans la détermination des peroxydes dans le lait.

Ce qui suit est la cascade de réactions chimiques qui, dans l'ordre de la quantité de peroxyde conduit à la formation de l'amylose bleu/ I complexe:

H₂O₂ → H₂O + O' ; 2I⁻ + H₂O + O' → 2OH⁻ + I₂; I₂+ I⁻ → I₃⁻; I₃⁻ + amilosio → complex blu I₃⁻/amylose

Si on exercice un excès stœchiométrique d'iodure et d'amylose par rapport à H₂O₂ l'intensité de la couleur bleue est corrélée avec la quantité d'iode présente, puis avec la quantité de H₂O₂ déposé sur la surface sensible.

À l'aide d'une échelle de couleur liée aux quantités croissantes de H₂O₂, une évaluation quantitative du peroxyde par unité de surface peut être trouvée.

Cette invention est décrite ci-dessous dans des exemples non limitatifs, se référant aux figure suivants :
Figure 1. L'appareil - L'appareil, qui est configuré comme une étiquette adhésive, est divisé en deux parties, la zone sensible de 20 cm² sur laquelle la variation chromatique est connectée à la quantité de peroxyde qui entre en contact avec la surface sensible et la zone de corrélation composée d'une échelle de couleur qui relie l'intensité de couleur avec des quantités de peroxyde.
Figure 2. Positionnement du dispositif d'invention dans un gros avion intercontinental comme le montre l'exemple 4.

### EXAMPLE

EXPLORE 1 - Chimie derrière le système de détection d'appareils pour surveiller les processus d'assainissement de l'environnement.

Le dispositif de l'invention vise à déterminer quels peroxydes quantitatifs sont entrés dans l'environnement pour être aseptisés sous forme atomisée, avec des particules comprises entre 0,3 et 0,5 m, aux quelles, dans les systèmes les plus sophistiqués, une charge de surface identique est transmise qui favorise la répulsion mutuelle, empêchant les phénomènes de coalescence. Le désinfectant atomique se comporte dans l'environnement pour être traité comme une masse pseudo-gaz qui se dilate dans tout l'espace disponible.

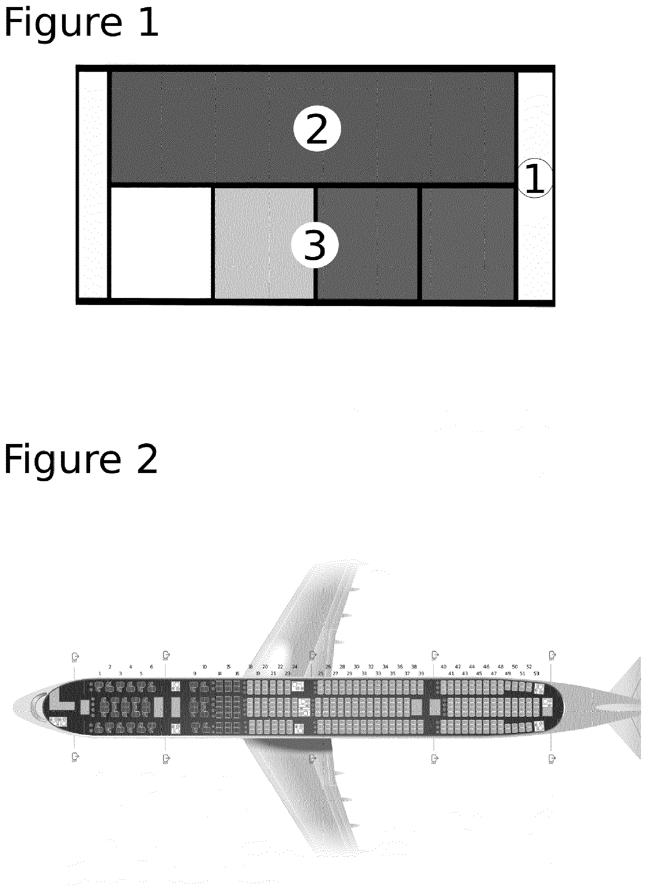

La vérification de l'efficacité du processus d'assainissement est liée au contrôle de la distribution uniforme du peroxyde dans l'environnement à traiter et à la réalisation sur les surfaces de quantités suffisantes de peroxyde par unité de surface pour assurer l'élimination quantitative de la charge virale contaminant. L'unité de détection (figure 1) est divisée en deux parties, la zone sensible de 20 cm² sur laquelle la variation chromatique est reliée à la quantité de peroxyde qui est entré en contact avec la surface sensible et la zone de corrélation composée d'une échelle de couleur qui relie l'intensité des couleurs avec des quantités de peroxyde. La surface sensible et l'échelle chromatique sont sur l'un des deux côtes d'une bande recto-verso, dont la deuxième face est utilisée pour faire adhérer l'unité de détection aux surfaces de l'environnement à aseptiser.

Ci-dessous se trouve la cascade de réactions chimiques qui, dans l'ordre, permet la détermination du peroxyde/quantitatif. Pour chaque quantité de peroxyde une taupe de I₂ est formé, qui en présence d'un excès de moi est causée par une taupe d'ion I, qui est situé dans la spirale de 6 molécules de glucose de l'origine à une taupe du complexe bleu I / amilosio. Cette séquence extrêmement rapide de réactions relie directement la quantité de peroxyde à l'intensité de la couleur bleue du complexe I/amilosio

H₂O₂ → H₂O + O' ; 2I⁻ + H₂O + O' → 2OH⁻ + I₂; I₂+ I⁻ → I₃⁻; I₃⁻ + amilosio → complex blu I₃⁻/amilose

Si vous utilisez dans un excès stœchiométrique d'iodure et d'amilosius par rapport à H₂O₂ l'intensité de la couleur bleue est corrélée avec la quantité d'iode présente, puis avec la quantité de H₂O₂ dépose sur la surface sensible.

À l'aide d'une échelle de couleur construite contre des quantités croissantes de H₂O₂, une évaluation quantitative du peroxyde par unité de surface peut être trouvée. Notez à partir de la littérature et des données expérimentales quelle est la quantité de peroxyde à laquelle exposer le virus pour l'inactiver, vous pouvez corréler l'intensité de la couleur bleue avec l'efficacité du processus d'assainissement.

### EXEMPLE 2- Description de l'appareil

Ce qui suit décrit la structure du dispositif de surveillance des processus d'assainissement de l'environnement, comme schématise dans la figure 1.

La surface sensible se compose de papier ou de tissu absorbant sur lequel un mélange d'amilosio et d'iodure est précédemment posé pour l'immersion dans une solution aqueuse de 10% amilosius et d'iodure de sodium à 4,6% suivie du séchage. La quantité d'amilosio/iodure de sodium fixée à 20 cm² de la surface sensible est d'environ 0,3 g calculée comme une différence de poids avant et après le chargement/séchage, qui correspond à 0,31 taupes de Nal/cm² et 0,62 taupes d'unités monoméries d'amilosi (glucose) ou 0,1e taupes de 6 unités de glucose correspondant à une spirale complète d'amilosio dans laquelle la molécule de I est insérée.

On sait que l'amilosio en présence de ione I₃⁻ forme un complexe stable de couleur bleue provenant de l'insertion de ione I₃⁻ I' dans la spirale de l'amilosius. Cette réaction est très spécifique parce que les molécules apparentées, telles que l'amylopectine, ne donnent pas cette réaction.

La spécificité élevée de la réaction permet d'être utilisé dans la construction de l'appareil non seulement amilosio purifié, mais aussi des sources moins chères d'amidons d'origine différente comme indiqué dans le tableau.

| **Source d'amidon de:** | **Amylose(%)** | **Amylopectine (%)** |
|---|---|---|
| Riz | 20-30 | 80-70 |
| Patate | 23-31 | 77-69 |
| Manioc | 16-25 | 84-75 |
| Blé | 30 | 70 |
| Maïs | 28 | 72 |
| Sorgho | 24-27 | 76-73 |

EXPLORE 3 - Processus industriel pour la préparation du dispositif de surveillance des processus d'assainissement de l'environnement.

Le processus industriel décrit ci-dessous ne doit pas être considéré comme limitant la taille et les types de matériaux utilisés. Les étapes de la préparation de l'appareil sont les suivantes: préparation de la surface active - Un ruban d'environ 2,5 cm de large de matériau absorbant d'environ 1 mm d'épaisseur (papier ou tissu ou tissu non tissé) est passé à vitesse constante dans une solution aqueuse de 10% d'amylose et d'iodure de sodium à 4,6% afin qu'il puisse être complètement imbibé, puis la bande passe dans un tunnel de séchage à l'air chaud (70-80 degrés C) pour le séchage complet. Le ruban séché d'environ 100 m de long est stocké enveloppé sur une bobine.

Active tape - Un ruban double à double face de 5 cm x 50 m est utilisé, avec de l'équipement automatique sur un visage collant des segments de 10 cm de ruban séché flanqué d'une bande plastique de 2,5 cm portant la séquence des couleurs de référence répétitivement tous les 10 cm.

Placer l'appareil sur les surfaces à désinfecter - à l'aide d'un dispositif portable qui vous permet de couper et d'adhérer à des segments de 10 cm de la bande active correspondant à l'ensemble de la séquence de couleurs de référence, l'opérateur procède à déposer l'appareil dans des endroits stratégiques de l'environnement à aseptiser.

EXPLORE 4 - Processus industriel pour la préparation d'une version simplifiée de l'appareil pour surveiller les processus d'assainissement de l'environnement.

Préparation de la surface active - La surface active est faite comme décrit dans l'exemple 3.

L'appareil - Le ruban de surface actif est collé sur une bande de 2,5 cm x 50 m de segments adhésifs et 10 cm sont coupés et fixés aux surfaces de l'environnement pour être aseptisés à l'aide d'un morceau de 1x1cm de ruban adhésif double. Des échantillons de la séquence de couleur de référence sont fournis aux travailleurs de l'assainissement et au personnel de conservation des données.

EXPLORE 5 - Processus industriel pour la préparation d'une version simplifiée supplémentaire de l'appareil pour la surveillance des processus d'assainissement de l'environnement.

Préparation de l'appareil - Les éléments 2,5 x 10 cm de matières absorbantes d'environ 2 mm d'épaisseur imprègnent complètement une solution aqueuse de 10 % d'amilosio et d'iodure de sodium à 4,6%, de sorte que l'appareil passe dans un tunnel de séchage à air chaud (70-80 degrés C) pour un séchage complet. L'appareil est fait pour adhérer aux surfaces de l'environnement pour être aseptisé par un morceau de ruban recto-verso de 1x1 cm. Des échantillons de la séquence de couleur de référence sont fournis aux travailleurs de l'assainissement et au personnel de conservation des données.

### EXEMPLE 6- Comment l'utiliser

Voici les étapes opérationnelles qui, dans l'ordre, permettent la surveillance du processus d'assainissement avec le dispositif de l'invention.
Étape 1 - Dépôt des systèmes de dosage dans des endroits stratégiques par le personnel d'assainissement.
Étape 2 - Sanitisation de l'environnement avec des peroxydes peroxydés atomisés respectant les temps liés à la taille de l'environnement, les temps de contact et la ventilation des espaces aseptisés.
Étape 3 - Vérifiez la justesse du processus d'assainissement, en comparant la couleur développée sur la bande sensible avec l'échelle de couleur pour voir si le seuil minimum requis a été dépassé.
Étape 4 - Récupérez toutes les étiquettes sensibles du personnel d'assainissement en appliquant une étiquette de 10 x 5 cm sur le côté coupe basse si l'appareil est du type décrit dans l'exemple 3.
Étape 5 - Préservation du matériel pour la documentation de l'efficacité du processus d'assainissement.

EXEMPLE 7- Stabilité de la réponse avec le temps d'exposition et le stockage de la découverte.

Vous contrôlez la stabilité au fil du temps de la couleur bleue sur les étiquettes exposées au processus d'assainissement. Même après 5 mois, la couleur bleue ne subit pas de changements significatifs.

EXEMPLE 8- Construction de l'échelle chromatique de référence.

Une solution aqueuse de H₂O₂ est préparée à 0,5% (5.000 ppm), 0,1% (1.000 ppm) et 0,05% (500 ppm). L'appareil est immergé dans la solution pendant 5 minutes comme décrit dans l'exemple 5 et la couleur développée est observée en reproduisant une copie fidèle pour être séquencée pour des concentrations croissantes de H₂O₂. Sur la base des données de la littérature, une coloration qui garantit une réduction complète de la charge virale doit être d'une intensité égale ou supérieure à celle développée avec 1000 ppm.

EXEMPLE 9 - Surveillance du processus d'assainissement d'un gros avion commercial à l'aide de peroxydes atomises.

Pour les avions commerciaux, l'assainissement avec des peroxydes atomises est la stratégie d'élection des opérateurs aériens et des entreprises d'assistance au sol qui fournissent des services de nettoyage et d'assainissement. Le traitement doit être effectue avant chaque vol le plus rapidement possible afin d'éviter les arrêts improductifs au sol à bord de l'avion. La désinfection, qui touche les cabines des passagers et de l'équipage, tient compte des recommandations de l'avionneur en termes d'agents désinfectants qui peuvent être utilisés et du type de surfaces en cause.

à cette fin, pour taus les désinfectants utilisés, en plus de leurs capacités de désinfection sur le SARS-CoV-2 spécifique, il est nécessaire de s'assurer que leur application n'a aucun effet préjudiciable sur la santé humaine ou aéronautique en termes de: structure de l'avion (par exemple la corrosion électronique et avionique (par exemple l'isolation du câble); capteurs (c.-à-d. détection de fumée); intérieurs (c.-à-d. installations, sièges, moniteurs , multimédias, fenêtres, cuisines, comptoirs). Dans cet esprit, les désinfectants atomisés H₂O₂ à des concentrations comprises entre 3 et 8% sont la meilleure solution possible.

Le nœud d'un processus d'assainissement est la documentation de l'efficacité sur toutes les surfaces de l'environnement traité. Il décrit le processus d'assainissement d'un gros aéronef, comme le BOEING 777-300ER (volume estime a 3xl0³ m³, 350 sièges), à l'aide d'une solution de H₂O₂ à 3% et 15% d'éthanol atomisé avec 3 SYSTEMS iHP d'INSTRUMENTS AM ou analogues Rami Health placé au centre de l'avion entre les lignes 8-9, 24-25, 39-40. Ce sont les temps de l'ensemble du cycle d'assainissement: la diffusion temporelle solution atomisée 250 minutes; temps de contact 120 minutes; Temps d'aération de 120 minutes, cycle global 490 minutes. Pour surveiller pleinement l'efficacité du processus d'assainissement de l'avion avant le début de l'assainissement, comme le montre la figure 2, 100 unités de détection selon l'appareil de l'invention sont placées. A la fin de l'assainissement, le personnel récupère les 100 unités, vérifiant la positivité du traitement sur chaque point. Les 100 unités montrent que la quantité de peroxyde qui est entré en contact avec la zone sensible a une intensité de couleur bleue équivalente et est compatible avec une réduction de la charge virale d'au moins 10⁵.

### Légende figure 1

1. Streep biadhesif
2. Zone de détection
3. Echelle chromatique

Unité de dispositif de détection selon l'invention. Sur un côté du streep double adhésif sont collés:
- Une zone sensible de 20 cm² composée d'une surface absorbante de papier, de tissu ou de tissu non tissé sur lequel est adsorbé un mélange d'amilosio et un iodide soluble dans l'eau.
- Une échelle chromatique plastique dans laquelle l'intensité de couleur bleue est relative à la quantité de peroxyde reçue par unité de surface.

### Légende figure 2

Positionnement des unités de dispositif de détection en fonction objet de l'invention. Il y a 100 unités de détection ainsi placées: 11 dans les toilettes; 16 en siège d'affaires (fichier 1, 5, 9 à gauche et à droite, 3 et 10 fauteuils centraux et compartiments supérieurs); 6 dans le siège économique premium (rangée 14 à gauche et à droite, 15 central sur les épaulettes et les frais généraux); 48 en siège économique (fichier 18, 23, 25, 29, 33, 39, 41, 45, 51 à gauche et à droite, 21, 27, 31, 35, 43, 49 central sur fauteuil et courroies de capuchon); 2 dans la cuisine avant; 4 dans le poste de pilotage; 24 tous les 5 mètres sur les couloirs de plancher.

## Revendications

1. Méthode de détection qualitative et quantitative du peroxyde d'hydrogène, présente par unité de surface, dans des environnements soumis à un processus d'assainissement à l'aide de composés peroxydiques aérosolisés/atomisés.

2. Méthode selon la revendication 1 basée sur la réaction colorimétrique qui se produit quand un peroxyde entre en contact avec un mélange d'iodure et d'amylose avec la formation d'une coloration bleue.

3. Méthode selon la revendication 2 dans laquelle ion d'iodure est liée à un cation qui garantit la solubilité dans l'environnement aqueux.

4. Méthode selon la revendication 2 dans laquelle l'amylose est pur ou en présence d'amylopectine.

5. Méthode selon la revendication 2 dans laquelle le rapport de poids entre amylose et iodure dans le mélange varie entre 2,6 et 10⁴.

6. Méthode selon les revendications de 2 à 5 dans lequel l'iodure est présente en forte excès par rapport au peroxyde de sorte que l'intensité de la coloration bleue dépend du réactif limitant, puis la quantité de peroxyde présent.

7. Méthode selon les revendications 2 à 6 ou la détermination quantitative du peroxyde présent est lue sur une échelle de couleur qui associe l'intensité de la couleur bleue avec la quantité de peroxyde qui a réagi par unité de surface.

8. Méthode selon les revendications de 1 à 7 dans lequel le mélange d'iodure/ amylose est: dissous dans un solvant; Absorbée sur un support; le support est séché; le support sèche est attache à un système de soutien.

9. Méthode selon la revendication 8 dans laquelle le mélange iodure/amylose est soluble dans l'eau.

10. Méthode selon la revendication 8 ou le Soutien se compose de papier essuie-tout ou de tissu non tissé.

11. Méthode selon la revendication 8 dans laquelle le système de support se compose d'un ruban adessif à double face, d'un côté le support sèche, l'autre côte est utilisé pour le faire adhérer dans l'environnement de mesure.

12. Dispositif pour effectuer la méthode telle que définie dans les claims 1 à 11, y compris le système de dosage de la quantité de peroxyde par unité de surface.

13. Appareil selon la réclamation 12 y compris: a) support sèche contenant le mélange d'iodure/amilosio soutenu sur un ruban à double face; échelle de couleur ou l'intensité de la couleur bleue est liée à la quantité de peroxyde; code numérique alpha pour identifier le lot de production et l'expirer.

14. Utilisation de l'appareil selon les revendications 12 et 13 pour exécuter la méthode telle que définie par les revendications 1 à 11.

15. Utilisation selon la revendication 14, y compris les étapes suivantes: le retrait du film protecteur de la face adhésive de la bande; l'observance de la bande dans des endroits stratégiques de l'environnement à assainir; identification du site de placement; contrôle de l' intensité e de la couleur développée à la fin du processus d'assainissement; récupération de la bande colorée; Couverture du visage adhésif étiqueté papier montrant les données d'identification du processus d' assainissement; dépôt de la bande dans le sachet de stockage.
